Europäisches Patentamt

**European Patent Office**

**Office européen des brevets**

(19)

(11) Publication number: **0 196 121**
**A1**

# EUROPEAN PATENT APPLICATION

(21) Application number: 86200238.3

(22) Date of filing: 18.02.86

(51) Int. Cl.⁴: **A61K 31/57** , A61K 31/58 ,
//(A61K31/58,31:57,31:71,31:60)

(30) Priority: 22.02.85 YU 276/85
14.02.86 YU 221/86

(43) Date of publication of application:
01.10.86 Bulletin 86/40

(84) Designated Contracting States:
AT BE CH DE FR GB IT LI LU NL SE

(71) Applicant: Galenika Pharmaceutical and Chemical
Industry
Zemun Batajnicki Drum B.B.
Beograd-Zemun(YU)

(72) Inventor: Visnjic, Pero, Dr.
M. Pijade 3
Makarska(YU)

(74) Representative: Noz, Franciscus Xaverius, Ir. et al
Algemeen Octrooibureau P.O. Box 645
NL-5600 AP Eindhoven(NL)

(54) Process for obtaining the preparation for the treatment of the disease psoriasis; drug for the treatment of psoriasis and its application.

(57) The process for obtaining the preparation used in the treatment of a skin disease psoriasis, whereby to a mixture of the components usually used for the preparation of, so called, greasy vehicle, a solution of the active ingredients being 9-fluoro-11-17,21-trihydroxy-16-methyl pregna-1,4-diene-3,20-dion-17,21-dipropionate and/or 6,9-difluoro-11,16,17,21-tetrahydroxy-pregna-1,4-diene-3,20-21-acetate-16,17-acetonide is added.

EP 0 196 121 A1

Process for obtaining the preparation for the treatment of the disease psoriasis; drug for the treatment of psoriasis and its application.

The invention refers to the obtaining of the preparation for the treatment of the skin disease Psoriasis manufactured in the form of medicinal ointment.

The aim of the invention is to obtain a medicinal agent based on corticosteroids, salicylates and antibiotics, using catalysts belonging to the group of highly purified proteins, which would clear the skin from psoriasis effectively, the most quickly and without endangering the integrity of healthy organs and skin regions and the relapses approximate to zero point.

It is well known that skin diseases are as old as human race and that people have been trying to find curative agents for them ever since. Therefore, plant extracts as well as drugs of both organic and nonorganic origin have been used.

Nowadays, there are about 2000 preparations used all over the world in the treatment of psoriasis most of which are based on corticosteroids, combinations of corticosteroids or combinations of antibiotics and salicylates.

The outstanding manufacturers of those preparations are Ciba-Geigy, Syntex, Diosynth, Schering, Upjohn etc. The main disadvantages of the mentioned preparations are secondary effects after the local application of corticosteroids, such as skin necrosis, steroidal dermatitis and, in some cases, secondary effects on the adrenal gland (e.g. suppression). The above disadvantages, namely, the activity of steroids on endocrine system are overcome by the preparation according to the invention.

This preparation desintegrates all kinds of crusts avoiding skin necrosis and, by neutralization of sugilation and subfusion processes, it induces vasconstrictive effects on peripheral blood circulation and, therefore, enhances recovery.

The adequate use of the drug, that is, the adequate combination of drug application and a certain hygienic-dietetic treatment as well as the maintenance of a certain state of mind (psychic peace) presuppose very rare relapses into illness.

The procedure for obtaining the drug according to the invention consists of the emulsion preparation consisting of oil phase, previously sterilized, and water, to which the active ingredients are added while constantly stirring and cooling, if necessary, so that all the phases are completed according to the order principle of ingredient stability to specified temperatures and after the addition of other components the homogenization and cooling processes are performed in order to obtain the ointment according to further aspects as disclosed in the claims.

The invention will be, in the procedure of the text, described through an example without the intention to be limited to it.

Example 1

The weighed components, usually used in the preparation of the, so called, greasy vehicle, are melted, while premantently homogenized, at 150 °C and, while stirring, cooled to 85 °C-90 °C so that the total preparation phase lasts for 20 minutes.

The filtered and melted phase is added, while constantly stirring, into demineralized water, previously sterilized for 10 minutes, after which the resulting emulsion is sterilized for 30 minutes at 85 °C-90 °C.

The emulsion is then cooled to 60 °C and the previously prepared solutions of the active ingredients are added while stirring. The solutions of the active ingredients are added in this order;

-9-fluoro-11-17,21-trihydroxy-16-methyl pregna-1,4-diene-3,20-dion-17,21-dipropionate

-6,9-difluoro-11,16,17,21-tetrahydroxy-pregna-1,4-diene-3,20-21-acetate-16,17-acetonide,

the ratio of the two corticosteroids being 1:1, after which, constantly stirring, are added gentamycin sulphate in the amount equivalent to at least 35000 IU/100 g of the ointment, vitamin A palmitate in the amount equivalent to 30000 IU/100 g of the ointment and, finally, 0.5 g of 2,4-dihydroxy-N-/3-hydroxy-propyl/-3,3-dimethylbutyramide.

After stirring and homogenization, salicylic acid is added in the amount representing 7% of 100 g of the ointment and the emulsion is then again subjected to homogenization and cooling.

During the manufacture of this preparation enough homogenizer should be provided for the component and vehicle preparation as well as for sterilization, vacuuming, micronization, that is for obtaining maximally homogenized mixture.

The corticosteroids used as the active ingredients should be dissolved in suitable solvents, by an aseptic procedure, at the temperature providing the stability of the dissolved substances.

The preparation, adequately applied and with addition of vitamins and with certain diet treatment, proved to be very effective not only in psoriasis cure but in significant decrease of the number of relapses as well.

**Claims**

1. The process for obtaining the preparation used in the treatment of a skin disease psoriasis, characterized in that to a mixture of the components usually used for the preparation of, so called, greasy vehicle, a solution of the active ingredients being 9-fluoro-11-17,21-trihydroxy-16-methyl pregna-1,4-diene-3,20-dion-17,21-dipropionate and/or 6,9-difluoro-11,16,17,21-tetrahydroxy-pregna-1,4-diene-3,20-21-acetate-16,17-acetonide is added.

2. Process according to claim 1, characterized in that the two active compounds are added in a ratio of 1:1.

3. Process according to claims 1 and 2, characterized in that gentamycin sulphate, vitamin A palmitate and about 0.5 g of 2,4-dihydroxy-N-/3-hydroxy-propyl/-3,3-dimethyl-butyramide are added as other active ingredients.

4. Process according to claim 3, characterized in that the gentamycin sulphate is added in the amount equivalent to about 35000 IU/100 g of the ointment and vitamin A palmitate is added in the amount equivalent to about 30000 IU/100 g of the ointment.

5. The drug used for the treatment of psoriasis, characterized in that it is obtained by the process according to the claims 1-4.

6. The drug according to the above claim which is applied in the treatment of the skin disease psoriasis.

7. The process for the preparation of an active part in a drug to be used according to claim 6, characterized in that the mixture of the compounds usually used for the preparation of, so called, greasy vehicle, homogenized and melted at about 150 °C, is cooled to 85 °C-90 °C, the whole preparation phase lasting for about 20 minutes, after which the so obtained oil phase is added, while constantly stirring, to demineralized water, previously sterilized, for about 10 minutes and the obtained emulsion sterilized for about 30 minutes, cooled to about 60 °C when the prepared solutions of the active ingredients are added to it while stirring so that all the phases are completed according to the order principle of ingredient stability to specified temperature and, finally, after the addition of salicylic acid in the amount representing about 7% of 100 g of the ointment, the emulsion is subjected to homogenization and cooling.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 116 444 (IMPERIAL CHEMICAL INDUSTRIES LTD.) * Page 6, line 19 - page 7, line 5; example 4 * | 1-4,7 | A 61 K 31/57 A 61 K 31/58 // (A 61 K 31/58 A 61 K 31:57 A 61 K 31:71 A 61 K 31:60 ) |
| X | GB-A-2 084 465 (GLAXO GROUP LTD.) * Page 1, lines 21-42 * | 1-4,7 | |
| X | UNLISTED DRUGS, vol. 27, no. 1, January 1975, page 5, Chatham, New Jersey, US. * Page 5, paragraph i: "Diprogenta" * | 5,6 | |
| X | DICTIONNAIRE VIDAL, 1974, pages 14,33, OVP, Paris, FR. * Page 14, paragraph: "Diprosone neomycine"; page 33, paragraph: "Topsyne neomycine" * | 5,6 | TECHNICAL FIELDS SEARCHED (Int. Cl.4) |
| X | DICTIONNAIRE VIDAL, 1974, pages 1699-1700, OVP, Paris, FR. * Pages 1699-1700, paragraph: "Topsyne gras" * | 5,6 | A 61 K |
| X | UNLISTED DRUGS, vol. 31, no. 2, February 1979, page 21, Chatham, New Jersey, US. * Page 21, paragraph d: "Belosalic" * | 5,6 | |
| | --- -/- | | |

The present search report has been drawn up for all claims

| Place of search | Date of completion of the search | Examiner |
|---|---|---|
| THE HAGUE | 23-05-1986 | BRINKMANN C. |

| | DOCUMENTS CONSIDERED TO BE RELEVANT | | Page  2 |
|---|---|---|---|
| Category | Citation of document with indication, where appropriate, of relevant passages | Relevant to claim | CLASSIFICATION OF THE APPLICATION (Int. Cl.4) |
| A | US-A-3 934 013  (B.J. POULSEN)<br><br>----- | 1-7 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 23-05-1986 | BRINKMANN C. |

EPO Form 1503 03 82